# EUROPEAN PATENT APPLICATION

(11) **EP 4 671 929 A1**
(43) Date of publication of application: **31.12.2025**
(21) Application number: 25184119.3
(22) Date of filing: 20.06.2025
(51) Int. Cl.: G06F 3/01, A61B 5/00, G06F 3/03, G06F 40/00, G06N 3/08, G06V 40/16, G10L 21/10

(54) **DEVICE AND METHOD FOR GENERATING AVATAR LIP-SYNC ANIMATION BASED ON MULTIMODAL BIOSIGNALS**

(30) Priority: 25.06.2024 KR 20240082346
(71) Applicant: Korea University Research and Business Foundation, Seoul 02841 (KR)
(72) Inventor: LEE, Seong Whan, 06292 Seoul (KR); PARK, Ji-Ha, 10115 Gimpo-si, Gyeonggi-do (KR); LEE, Seo Hyun, 06007 Seoul (KR)
(74) Representative: Jung, Minkyu

(57) **Abstract**

The present disclosure relates to a device and method for generating avatar lip-sync animation based on multimodal biosignals, The device comprises a multimodal data collection unit configured to collect data including biosignal data including brain waves when a user imagines speaking and image data; a preprocessing unit configured to preprocess the multimodal data; a feature extraction unit configured to extract feature vectors including the user's biosignal feature and facial feature from the preprocessed multimodal data; an avatar generation unit configured to generate an avatar; a lip-sync reconstruction unit configured to predict the mouth shape and facial movement when the user imagines speaking by inputting the extracted feature vectors to a pre-prepared lip-sync reconstruction model; and a lip-sync animation implementation unit for implementing an avatar lip-sync animation by applying the mouth shape and facial movement predicted by the lip-sync reconstruction unit to the avatar generated by the avatar generation unit.

## Description

### CROSS REFERENCE TO RELATED APPLICATIONS

This application claims priority to Korean Patent Application No. 10-2024-0082346, filed on June 25, 2024, in the Korean Intellectual Property Office, which is incorporated by reference herein in its entirety.

### TECHNICAL FIELD

The present disclosure relates to a device and method for generating avatar lip-sync animation based on multimodal biosignals, and more specifically, to a device and method for generating avatar lip-sync animation based on multimodal biosignals that can generate an avatar corresponding to a user's facial image and implement avatar lip-sync animation based on the multimodal biosignals when the user imagines speaking using a pre-prepared lip-sync reconstruction model.

### BACKGROUND ART

Brain-Computer Interface (BCI) is a technology that directly connects neurological signals of the brain to a computer system thereby enabling communication and control.

To this end, various biosignal measurement technologies are used to identify brain activities such as the user's thoughts, concentration and imagination and convert them into digital instructions.

The brain-computer interface becomes a very important tool, especially for people with limited athletic ability, and allows them to perform activities such as using computers, moving robotic arms and even controlling wheelchairs, etc.

This technology provides a new way of interaction in various fields, including virtual reality, video games, neuroscience research, and even art and music creation.

Recently, the brain-computer interface technology has become more sophisticated along with the development of algorithms that interpret brain signals, and this has the potentiality capable of innovatively changing the interaction between human and machines in the future.

Meanwhile, researches have been being recently conducted on a method of implementing a speaking human face through lip-sync between a face synthesized with computer graphics and a human voice.

As a prior art, 'Voice-based Automatic Lip-sync Animation Device and Method and Recording Medium' was proposed in Korean Laid-open Patent Publication No. 10-2006-0031449 (published on April 12, 2006).

Existing lip-sync animation technologies, including the above-mentioned prior art were mainly based on methods of reconstructing the shape of mouth by receiving voice data.

However, existing methods of generating a speaking face through lip-sync animation necessarily require the use of recorded voice data spoken directly by the user. Therefore, there were the problems that existing systems could not utilize voice data for patients who have difficulty to speak or in quiet situations, and there were the limitations that the systems could not express detailed emotions such as the user's facial expressions and nuances.

Meanwhile, as another prior art, 'Brain-Computer Interface System and User Conversation Intention Recognition Method using the same' was proposed in Korean Laid-open Patent Publication No. 10-2020-0052807 (published on May 15, 2020).

However, brain-computer interface-based communication systems including the above-mentioned another prior art have been mainly implemented in a manner of passively reading and communicating user's intentions such as simple class classification or sentence generation using brain waves during the speaking.

Recently, communication systems utilizing brain signals have been developed a lot in the field of brain-computer interface, and various methodologies have been being developed by being grafted into the field of artificial intelligence.

Among them, user communication technology based on speaking imagination has the advantage of capable of communicating user's intentions without the user's direct speaking.

However, user communication technology through the brain-computer interfaces has limitations such as low real-time decoding performance, low recognition rate, and still difficulty in in achieving understandable level of voice synthesis.

Various methods have been proposed to improve performance, but the technology that communicates intentions by invasive brain wave measurement is expensive and difficult to use in real life, and it is of little use as a method that recommends surgery to the general public.

A technology that synthesizes voices using brain waves during speaking is also being developed, but this has limitations in that it is restrictive to utilize it for patients who have difficulty to speak or in quiet environments where it is not allowed to speak.

Accordingly, there is a need to develop a new technology that can output avatar lip-sync animation by receiving biosignals during speaking imagination rather than learning brain waves and recorded voice data at the time of speaking.

### SUMMARY

The present disclosure has been created to overcome the limitations of the conventional technologies and to meet the demand for new technology development, and the purpose of the present disclosure is to provide multimodal biosignal-based avatar lip-sync animation generation device and method thereof that is capable of receiving biosignals including brain waves when a user imagines speaking and outputting avatar lip-sync animation based on them.

In order to achieve the above-mentioned purpose, the multimodal biosignal-based avatar lip-sync animation generation device according to the present disclosure comprises a multimodal data collection unit configured to collect multimodal data including biosignal data which includes brain waves when a user imagines speaking and image data; a preprocessing unit configured to preprocess the multimodal data; a feature extraction unit configured to extract feature vectors including the user's biosignal feature and facial feature from the preprocessed multimodal data; an avatar generation unit configured to generate an avatar that represents the user's appearance; a lip-sync reconstruction unit configured to predict the mouth shape and facial movement when the user imagines speaking by inputting the extracted feature vectors to a pre-prepared lip-sync reconstruction model; and a lip-sync animation implementation unit configured to implement an avatar lip-sync animation by applying the mouth shape and facial movement predicted by the lip-sync reconstruction unit to the avatar generated by the avatar generation unit.

The avatar generation unit is configured to generate an avatar in a 2D or 3D form from the user's image data using computer vision technology, and maps the user's facial feature extracted by the feature extraction unit to the generated avatar to thereby specify a facial landmark; and the lip-sync animation implementation unit is configured to implement an avatar lip-sync animation by applying the mouth shape and facial movement predicted by the lip-sync reconstruction unit to the avatar generated by the avatar generation unit, based on the coordinate values of the facial landmark.

The multimodal biosignal-based avatar lip-sync animation generation device according to the present disclosure further comprises a feature convergence unit configured to converge the feature vectors extracted from the feature extraction unit and converting them into an embedding convergence vector, wherein the lip-sync reconstruction unit is configured to predict mouth shape and facial movement when the user imagines speaking by inputting the embedding convergence vector to a pre-prepared lip-sync reconstruction model.

The multimodal data collection unit includes a presented sentence transfer display module configured to transfer a presented sentence for speaking imagination to the user; a biosignal collection module configured to collect biosignal data by measuring biosignals including the user's brain waves; an image collection module configured to collect image data by capturing a facial image of the user; and a data storage module configured to store the biosignal data of the user who imagines speaking in response to the transferred presented sentence and the image data, together with a trigger value being recorded over time.

The biosignal collection module further includes an electromyography in the measured biosignal of the user; and the lip-sync reconstruction unit is configured to predict the mouth shape and facial movement by inferring an articulatory organ movement trajectory corresponding to the speaking imagination based on the electromyography.

The feature convergence unit applies a weight based on a predetermined standard to the feature vectors extracted by the feature extraction unit, converges the feature vectors to which the weight has been applied and converts them into an embedding convergence vector.

The lip-sync reconstruction model is configured of any one of: a first prediction model configured to predict the mouth shape and facial movement when the user imagines speaking from the extracted feature vectors, or a second prediction model configured to identify and classify the user's intentions from the extracted feature vector, and predicting the mouth shape and facial movement when the user imagines speaking based on the classified intention.

A multimodal biosignal-based avatar lip-sync animation generation method according to the present disclosure comprises a multimodal data collection step of collecting multimodal data including biosignal data which includes brain waves when a user imagines speaking and image data; a preprocessing step of preprocessing the multimodal data; a feature extraction step of extracting feature vectors including the user's biosignal feature and facial feature from the preprocessed multimodal data; an avatar generation step of generating an avatar that represents the user's appearance based on facial features among the extracted feature vectors; a feature extraction step of extracting feature vectors including the user's biosignal feature and facial feature from the preprocessed multimodal data; an avatar generation step of generating an avatar that represents the user's appearance based on the facial feature among the extracted feature vectors; a lip-sync reconstruction step of predicting the mouth shape and facial movement when the user imagines speaking by inputting the extracted feature vectors to a pre-prepared lip-sync reconstruction model; and a lip-sync animation implementation step of implementing an avatar lip-sync animation by applying the mouth shape and facial movement predicted in the lip-sync reconstruction step to the avatar generated in the avatar generation step.

A multimodal biosignal-based avatar lip-sync animation generation method according to the present disclosure further comprises a feature convergence step of converging the feature vectors extracted in the feature extraction step and converting them into an embedding convergence vector, wherein the lip-sync reconstruction step is configured to predict the mouth shape and facial movement when the user imagines speaking by inputting the embedding convergence vector to a pre-prepared lip-sync reconstruction model.

By the above configuration, the device and method for generating a multimodal biosignal-based avatar lip-sync animation according to the present disclosure have the advantage of being able to identify the user's intention from the biosignal when the user imagines speaking and provide it as an avatar lip-sync animation.

In addition, the device and method for generating a multimodal biosignal-based avatar lip-sync animation according to the present disclosure can be developed into a system without restrictions on various uses by utilizing biosignals including non-invasive speaking imagination brain waves, and can enable lip-sync animation that visually transfers the user's intention without the user directly speaking out loud by extracting speaking and facial reconstruction information contained in the biosignals, and can express and transfer the user's emotions and intentions through facial expressions, and can promote future-oriented technology by enabling realistic and dynamic communication in the next-generation digital world by utilizing avatars.

### DETAILED DESCRIPTION OF THE DRAWINGS

Figure 1 is a configuration diagram of a multimodal biosignal-based avatar lip-sync animation generation device according to an embodiment of the present disclosure.
Figure 2 is a configuration diagram of a multimodal data collection unit according to an embodiment of the present disclosure.
Figure 3 is an example diagram of an avatar generation unit according to an embodiment of the present disclosure.
Figure 4 is a data collection and processing flow diagram for lip-sync reconstruction according to an embodiment of the present disclosure.
Figure 5 is a data processing flow diagram for implementing avatar lip-sync animation according to an embodiment of the present disclosure.
Figure 6 is a flow diagram of a multimodal biosignal-based avatar lip-sync animation generation method according to an embodiment of the present disclosure.

### DETAILED DESCRIPTION OF THE DISCLOSURE

Hereinafter, a multimodal biosignal-based avatar lip-sync animation generation device and method according to the present disclosure will be described in more detail with reference to the embodiments illustrated in the drawings.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," "circuit" or "circuitry". A module may be a single integral component, or a minimum unit or part thereof, adapted to perform one or more functions. For example, according to an embodiment, the module may be implemented in a form of an application-specific integrated circuit (ASIC).

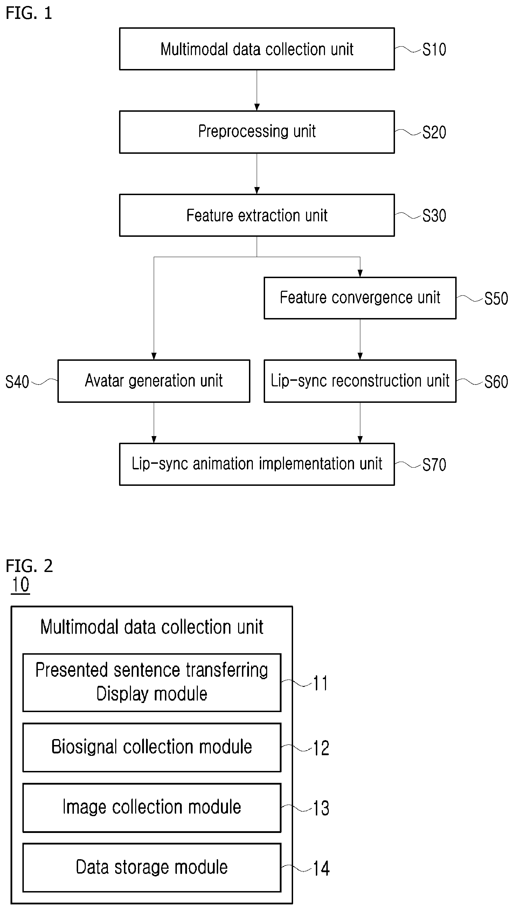

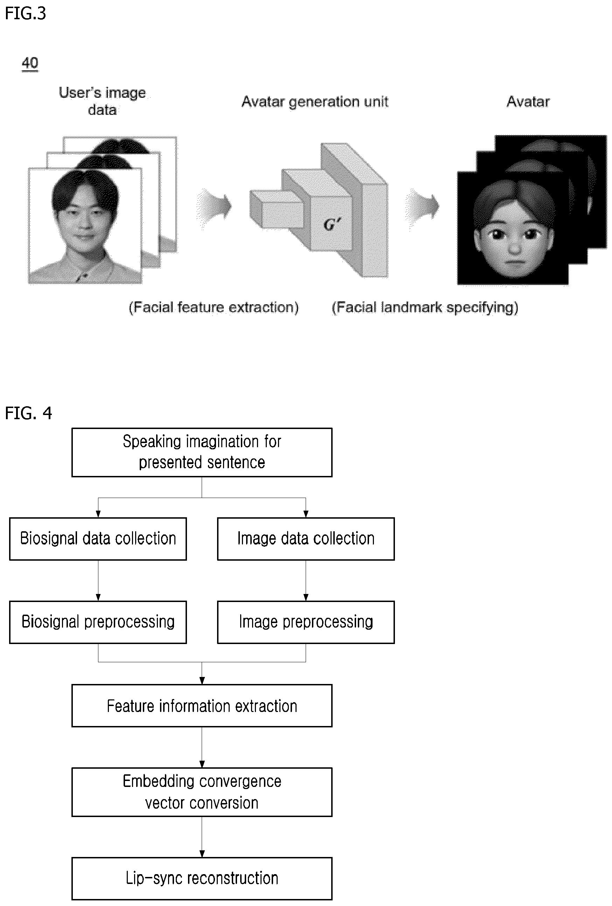

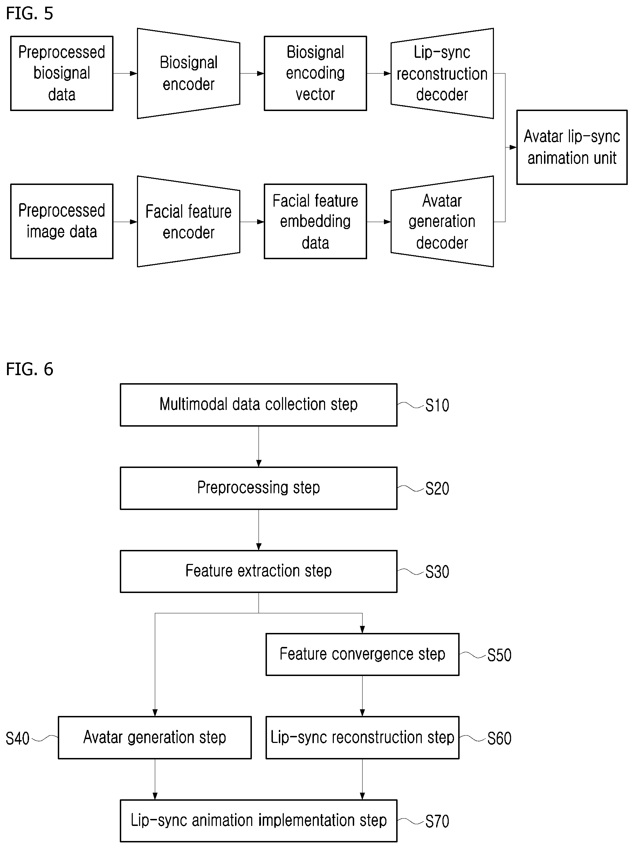

FIG. 1 is a configuration diagram of a multimodal biosignal-based avatar lip-sync animation generation device according to an embodiment of the present disclosure, FIG. 2 is a configuration diagram of a multimodal data collection unit according to an embodiment of the present disclosure, FIG. 3 is an exemplary diagram of an avatar generation unit according to an embodiment of the present disclosure, FIG. 4 is a data collection and processing flow diagram for lip-sync reconstruction according to an embodiment of the present disclosure, and FIG. 5 is a data processing flow diagram for implementing an avatar lip-sync animation according to an embodiment of the present disclosure.

Referring to FIG. 1, the multimodal biosignal-based avatar lip-sync animation generation device according to an embodiment of the present disclosure comprises a multimodal data collection unit 10, a preprocessing unit 20, a feature extraction unit 30, an avatar generation unit 40, a feature convergence unit 50, a lip-sync reconstruction unit 60 and a lip-sync animation implementation unit 70.

The multimodal data collection unit 10 is configured to collect multimodal data including biosignal data such as brain wave and electromyogram when the user imagines speaking, and image data such as the user's facial image.

In one embodiment of the present disclosure, the above multimodal data collection unit 10 may include a presented sentence transfer display module 11, a biosignal collection module 12, an image collection module 13, and a data storage module 14 as shown in FIG. 2.

The presented sentence transfer display module 31 is configured to transfer a presented sentence for speaking imagination to the user through a screen.

In one embodiment of the present disclosure, the presented sentence transfer display module 31 may be configured to transfer a guideline image including a standard speaking lip shape for the presented sentence to the user.

The biosignal collection module 12 is configured to collect biosignal data by measuring biosignals such as the user's brain wave and electromyogram.

Brainwave or Electroencephalography (EEG) as a biosignal refers to the electrical activity of the brain measured through electrodes attached to the scalp. This signal is used as an important tool for understanding the various states and activities of the brain, and in particular, has the advantage of being able to precisely track functional changes in the brain in time. The brainwave is widely used in neuroscience research, clinical diagnosis, neuropsychology, brain-computer interface development and the like. In particular, in the field of brain-computer interface, the user's intentions or thoughts are recognized and converted into machine instructions, which allows the user to control external devices with just imagination, thereby providing a new method of communication and interaction. This brainwave signal utilization technology can be applied to complicated tasks such as real-time lip-syncing of digital avatars or animations by analyzing brain activity patterns related to speaking imagination.

The biosignal collection module 12 for measuring the user's brainwave can be configured as a wearable, non-invasive device for speaking imagination-based brainwave measurement, and as an embodiment, it can be configured to measure real-time brain wave data depending on the user's biosignal with a cap-shaped device, to which a total of 128 electrodes are attached, worn outside of the scalp. A gel-type conductive material is applied to the scalp to match the electrodes so that the brain waves can be measured well.

At this time, the biosignal collection module 12 for measuring the user's brain waves also measures speaking attempt-based brainwave for analysis and comparison of speaking imagination-based brain waves, and measures the brain waves when only the mouth shape moves without sound. The measured brain waves are recorded along with the trigger value and the taken time. The brain wave data is stored in the specified database path of the data storage module 14 described below, and it is desirable to back up the data to an external storage device for data preservation.

Meanwhile, electromyography (EMG) as a biosignal refers to an electrical signal related to muscle activity, and measures electrical changes depending on muscle contraction and relaxation. This signal plays an important role in evaluating the functional status of muscles and the health of the nervous system, and is widely used in medical diagnosis, rehabilitation treatment, sports science, and biomechanics research. In particular, the EMG measurement technology is very useful for research related to muscle control by precisely monitoring muscle activity. It can be used to integrate natural movements of the human body into digital avatars or robot technology, and can reconstruct facial movements or facial expression changes based on specific muscle movements of the user. It can be utilized in various fields such as an intuitive communication system that synthesizes voice based on articulatory muscle movements of a speaking situation.

The EMG measured as described above allows to infer the articulatory kinematic trajectories (AKTs) corresponding to speaking imagination based on the EMG in the lip-sync reconstruction unit 60 described below, thereby predicting the mouth shape and facial movements.

Here, the AKTs are information that have precisely recorded the articulatory organ movement during the speaking process. They play an important role in understanding how the articulatory organs such as the lips, tongue and jaw move and produce sounds. By analyzing the articulatory kinematics, it is widely used in the fields of linguistics, phonetics, medicine, and computer science. For example, the AKTs can be used to analyze the articulation patterns of people with speaking disorders and develop treatment methods for them. In addition, combined with artificial intelligence technology, it is also significantly applied to real-time lip-sync animation, sophisticated avatar expression, and improving the accuracy of voice recognition system. The articulatory kinematic trajectory information plays an essential role in deepening the understanding of the human speaking process by providing highly detailed articulation data and in developing more natural and realistic communication technology based on this.

The image collection module 13 is configured to collect image data by capturing the user's facial image.

The image collection module 13 records the user's facial image while attempting to speak and imagining to speak using a camera attached to the display screen. The image collection module 13 records moving and still images in real time, and records the same trigger value over time to match the brain wave data recording. The facial image data is stored in the specified database path of the data storage module 14, and it is desirable to back up it to an external storage device for data preservation.

The data storage module 14 is configured to store the biosignal data and the image data of the user who imagines to speak in response to the transmitted presented sentence, along with the trigger value recorded over time.

The preprocessing unit 20 is configured to preprocess the multimodal data.

As an example, preprocessing of brainwave data recorded over a continuous period of time among the above multimodal data can sort them into the trigger value recorded together, and separate only brainwaves during actual attempt to speak and imagination for about 1.5 seconds, obtain and store information on frequency values in a two-dimensional form of time and channel regarding the relevant portion.

The feature extraction unit 30 is configured to extract a feature vector including the user's biosignal feature and facial feature from the preprocessed multimodal data.

The biosignal feature may include a brainwave feature and an EMG feature, and the facial feature may include a facial shape feature, a mouth shape feature and a facial movement feature.

The feature extraction unit 30 generally uses a common spatial pattern analysis (CSP) or a linear discriminant analysis (LDA) to extract the feature vectors. It is used to sort characteristics such as movement from the biosignal well and to obtain information about facial features and landmark features from facial image data well, and the extracted feature vectors are utilized in learning an artificial intelligence neural network model, which is advantageous in achieving high prediction performance.

As an example, the feature extraction unit 30 may be configured to extract a phoneme-specific feature and mouth shape-specific movement feature vectors from the speaking imagination brainwave in the case of brainwave feature, extract a facial feature vector in the case of facial feature, and inter-converge the phoneme-specific and mouth shape-specific movement feature vector and the facial feature vector in the feature convergence unit 50 described below so that the vectors can be converted into an embedding convergence vector.

The avatar generation unit 40 is configured to generate an avatar that represents the user's appearance as shown in FIG. 3.

The avatar refers to a virtual character or image that functions as a representative of a user or a spokesperson for the user in the digital world. It mainly refers to a digital agent that reflects the user's physical, emotional, or individual characteristics in online game, virtual reality and social media. The avatar has various appearances and characteristics that allow users to express their own unique identity and individuality as a means of expressing and interacting with themselves in a digital environment. Recently, with the developments of artificial intelligence technologies, there is a trend in which avatars imitate the user's real-time movements, facial expressions, and even speaking to add realism. These developments enrich the experience of virtual reality and augmented reality, and open up new possibilities in the fields of online communication and interactive entertainment.

In one embodiment of the present disclosure, the avatar generation unit 40 is configured to generate a 2D or 3D avatar from the user's image data using computer vision technology, and map the user's facial feature, especially facial shape feature, extracted from the feature extraction unit 30 to the generated avatar to thereby specify facial landmarks.

The facial landmarks, which are points representing the main features of the face, are widely used in the fields of computer vision research and image processing. These landmarks refer to points corresponding to important parts of the face such as the eyes, nose, mouth and jawline, and they play a key role in various applications such as facial recognition, emotion analysis, facial tracking, lip-syncing and avatar generation. Accurately identifying and tracking facial landmarks is essential to understand and interpret the shape and movement of the face, and through this, even subtle facial expression changes and eye movements can be captured. Recently, with the developments of artificial intelligence technologies, more precise and faster facial landmark detection has become possible, which enables innovative applications in various fields such as virtual reality, augmented reality, interactive games and security systems.

The avatar generation unit 40 can utilize open sources for generating avatars, and there are examples of avatars such as Apple's Memoji. It can also be applied to other character avatars, not just the user's personal face, and in this case, correspondence of landmark coordinates between the user's facial landmark and the new character avatar is necessary.

The facial landmark specified in the above avatar generation unit 40 becomes a coordinate value standard in the lip-sync animation implementation unit 70 described later, and a detailed description thereof will be described later.

The feature convergence unit 50 is configured to converge the feature vectors extracted from the feature extraction unit 30 and convert them into an embedding convergence vector.

In addition, in another embodiment, the feature convergence unit 50 may be configured to apply a weight based on a predetermined standard to the feature vectors extracted from the feature extraction unit 30 and to converge the feature vectors to which the weight has been applied and convert them into an embedding convergence vector.

The weight application standard may be based on user's selection or multimodal data with high importance may be set in advance.

The lip-sync reconstruction unit 60 is configured to predict the mouth shape and facial movement when the user imagines speaking by inputting the extracted feature vectors into a pre-prepared lip-sync reconstruction model.

In one embodiment of the present disclosure, the lip-sync reconstruction unit 60 can be configured to predict the mouth shape and facial movement when the user imagines speaking by inputting the embedding convergence vector in which the feature is converged by the feature convergence unit 50 into a pre-prepared lip-sync reconstruction model.

This lip-sync reconstruction model can be implemented as a deep learning model or an auto encoder such as a deep neural network (DNN), a convolutional neural network (CNN), a recurrent neural network (RNN).

In addition, in one embodiment of the present disclosure, the lip-sync reconstruction model can be configured as either a first prediction model that is configured to predict the mouth shape and facial movement when the user imagines speaking from the extracted feature vectors or a second prediction model that identifies the user's intentions from the extracted feature vectors, classifies them and predicts the mouth shape and facial movement when the user imagines speaking based on the classified intentions.

Meanwhile, the lip-sync reconstruction unit 60 can be configured to adopt only the user's brain waves among the biosignals measured during the user's speaking imagination as an input value or to adopt other biosignals together with the user's brain waves as multiple input values.

As an example, in the case of electromyography as the other biosignals, the lip-sync reconstruction unit 60 may be configured to predict the mouth shape and facial movement by inferring the articulator organ kinetics trajectories corresponding to the speaking imagination based on the electromyography.

At this time, when other biosignals such as electromyography together with the user's brain waves are adopted as multiple input values, the feature convergence unit 50 can be configured to apply a weight, which corresponds to the importance of the biosignal adopted as an input value or to the user's setting value, to the feature vectors extracted from the feature extraction unit 30, and to converge the feature vectors to which the weight has been applied and convert them into an embedding convergence vector, and the lip-sync reconstruction unit 60 can be configured to predict the mouth shape and facial movement corresponding to the speaking imagination by inputting the embedding convergence vector converged with the feature vector to which the weight has been applied.

FIG. 4 illustrates a data collection and processing flowchart for lip-sync reconstruction according to an embodiment of the present disclosure.

Hereinafter, with reference to Fig. 4, an avatar generation and lip-sync animation methodology for visually transferring a user's intention from non-invasive brain wave data during speaking imagination will be described.

A speaking avatar can be implemented by reconstructing the mouth shape and facial movement from speaking imagination brain wave data for lip-sync animation and applying it to a newly generated avatar from the user's facial image data. Compared to the existing speaking imagination-based brain computer interface technology mentioned above, this technology can regenerate a combination of mouth shapes corresponding to all human speaking sounds by decoding only about 15 vismes representing mouth shapes and thus, it can configure moving mouth shapes for sentences that have not been determined before, thereby having greater expandability. In addition, by utilizing the features related to about 15 vismes, the performance of regeneration is also higher than that of existing brain wave-based systems.

The speaking imagination brain wave performs both the speaking attempt situation and the speaking imagination attempt while wearing a brain wave measurement device. In order to additionally use in the brain wave data learning for speaking imagination, the speaking attempt situation is recorded at the same time, and only the mouth shape is moved without making a sound to assist the feature information for the word or sentence to speak. Learning about the phoneme units that make up a word or sentence is performed first, and the speaking attempt situation and the speaking imagination attempt are each performed for about 1.5 seconds. In addition, the actual mouth shape classes when actually speaking a word or sentence are defined, and learning for the mouth shape unit is additionally performed, in which the speaking attempt situation and the speaking imagination attempt are each performed identically for the same time. This process is repeated n times to collect speaking imagination brain wave data for each user.

A total of 128 channels are arranged to measure brain waves, including the Broca-Wernicke area where speaking imagination brain waves are characteristically expressed. In general, brain wave data is measured by collecting the sampling frequency at a frequency of 1,000 Hz, in which this value may vary depending on the situation. During the measurement of brain wave data, eye blinks or unintended unnecessary muscle movements, etc. may be recorded as noise, and the noise is removed through a preprocessing process using the preprocessing unit 20. Brain wave data depending on time is sorted based on different trigger values, and learnable data can be collected using this. The brain wave data finally obtained is extracted using a time-frequency feature.

At the same time, while measuring speaking imagination brain wave data, the user's face is recorded using a camera placed in front of the user, and the user's facial feature and image data are acquired. The relevant system can be implemented by utilizing the relevant image information or by using only one 2D photo provided directly by the user later. Information on the mouth shape and facial movement in the speaking attempt situation can be obtained, and this is used as an additional feature in the learning process. About 68 facial landmarks can be obtained from the user's face recorded in the facial image data, which are then used to apply lip-sync animation to the avatar.

The preprocessed data is converted into feature vectors that can express the mouth shape and facial movement. The brain wave data is converted into a biosignal embedding vector through a biosignal encoder, and the image data is converted into a facial feature embedding vector through a facial feature encoder. The relevant embedding vectors are then stored in the form of a converged vector for subsequent learning, and the relevant embedding vector conversion process serves as a process to better represent the mouth shape feature of the phoneme and speaking sound of the speaking imagination, and enables high-quality reconstruction by sorting facial features well.

The information on the articulatory organ movements for implementing lip-sync from the brain wave data is analyzed. Based on the previous research results that the articulatory organ movement trajectories are encoded in the speaking sensorimotor cortex of the brain, the independent user's articulatory organ movement can be inferred. The 12-dimensional articulatory organ movement trajectories can be analyzed, which corresponds to the x, y displacement values for various parts of the tongue and the upper and lower parts of the lip.

The converted embedding vector is learned through the lip-sync reconstruction model and the results for the mouth shape or facial movement are derived. The lip-sync reconstruction model for learning adjusts parameters such as the learning rate and batch size to thereby allow to achieve the optimal learning results.

The movement change re-arranges the values of the positions in the form of the trajectory of the facial landmarks and reconstructs the relevant landmarks, thereby implementing the complex speaking facial lip-sync animation. The generated avatar has coordinates corresponding to N (N>10) landmarks and the coordinates are applied to fit the movement of the reconstructed landmarks so as to output a natural avatar lip-sync animation.

The relevant process is based on the model learned with the collected user-customized data. When the user uses the system, brain waves and biosignals are given to the pre-learned model in real time, and the moving facial synthesized based on the given biosignals is output on the screen in real time. The user's avatar outputted at this time can be selectively applied in the way the user wants by utilizing a personalized avatar generated from the user's facial image data or a 2D photo provided by the user.

The lip-sync animation implementation unit 70 is a configuration to implement avatar lip-sync animation by applying the mouth shape and facial movement predicted by the lip-sync reconstruction unit to the avatar generated by the avatar generation unit.

More specifically, the lip-sync animation implementation unit 70 can implement avatar lip-sync animation by applying the mouth shape and facial movement predicted by the lip-sync reconstruction unit 60 based on the coordinate values of the facial landmarks to the avatar generated by the avatar generation unit 40.

FIG. 5 illustrates a data processing flow chart for implementing avatar lip-sync animation according to an embodiment of the present disclosure.

Referring to Fig. 5, preprocessed biosignal data is passed through a biosignal encoder, a biosignal embedding vector and a lip-sync reconstruction decoder to generate a mouth shape and facial movement when the user imagines speaking, and the preprocessed image data is passed through a facial feature encoder, a facial feature embedding vector and an avatar generation decoder to generate an avatar, and the generated mouth shape and facial movement and the avatar are mutually combined to implement a final avatar lip-sync animation.

The lip-sync animation implementation unit 70 can realistically implement the information derived from the lip-sync reconstruction unit 60 based on the landmark coordinate values of the generated avatar. The lip-sync animation implementation unit can allow the avatar to lip-sync as if saying the presented sentence exactly as imagined by the user and output its actual voice together to thereby implement an avatar animation that seems to actually speak. The lip-sync animation implementation unit can be configured to receive feedback by displaying the result on the screen in real time so that the user can see the result and prove that his or her communication is accurate.

In the above, the device configured to generate a multimodal biosignal-based avatar lip-sync animation according to the present disclosure has been discussed, and a method for generating a multimodal biosignal-based avatar lip-sync animation according to the present disclosure will be described below.

FIG. 6 is a flowchart of a method for generating a multimodal biosignal-based avatar lip-sync animation according to an embodiment of the present disclosure.

Referring to FIG. 6, the method for generating a multimodal biosignal-based avatar lip-sync animation according to an embodiment of the present disclosure comprises a multimodal data collection step (S10), a preprocessing step (S20), a feature extraction step (S30), an avatar generation step (S40), a feature convergence step (S50), a lip-sync reconstruction step (S60) and a lip-sync animation implementation step (S70).

Since the specific details of each step have been described in detail in the description regarding the device configured to generate a multimodal biosignal-based avatar lip-sync animation according to the present disclosure, only the features of each step will be briefly described below.

The multimodal data collection step (S10) is a step of collecting multimodal data including biosignal data and image data including brain waves when the user imagines speaking.

The preprocessing step (S20) is a step of preprocessing the multimodal data.

The feature extraction step (S30) is a step of extracting feature vectors including the user's biosignal feature and facial feature from the preprocessed multimodal data.

The above avatar generation step (S40) is a step of generating an avatar that represents the user's appearance based on the facial feature among the extracted feature vectors.

The feature convergence step (S50) is a step of converging the feature vectors extracted in the above feature extraction step and converting them into an embedding convergence vector.

The lip-sync reconstruction step (S60) is a step of predicting the mouth shape and facial movement when the user imagines speaking by inputting the extracted feature vectors to a pre-prepared lip-sync reconstruction model.

In one embodiment of the present disclosure, the lip-sync reconstruction step (S60) may be configured to predict the mouth shape and facial movement when the user imagines speaking by inputting the embedding convergence vector in which the features have been converged in the feature convergence step (S50) to a pre-prepared lip-sync reconstruction model.

The lip-sync animation implementation step (S70) is a step of implementing an avatar lip-sync animation by applying the lip shape and facial movement predicted in the lip-sync reconstruction step (S60) to the avatar generated in the avatar generation step (S40).

The multimodal biosignal-based avatar lip-sync animation generation device and method according to the present disclosure, which have the configurations as described above, provide innovative applicability in the fields of medical care, assistive devices and daily communication. In particular, as a customized solution for individuals with physical or linguistic limitations, it enables them to directly and efficiently express their intentions. This technology can be integrated with various interfaces such as voice generation devices, virtual and augmented reality systems, and next-generation communication systems, and can be utilized to control various digital devices by interpreting the user's thoughts in real time.

This brain-computer interface technology can also serve as a rehabilitation training tool for patients with stroke or severe muscle damage, and will be a major turning point in health care and in overcoming disabilities. Furthermore, the present technology can be applied to general public markets such as education, entertainment and personal productivity enhancement, and thus it is expected to be deeply integrated into everyday life so that anyone can use it with future technological advancements.

The multimodal biosignal-based avatar lip-sync animation generation device and method described above and illustrated in the drawings are only one embodiment for implementing the present disclosure, and should not be construed as limiting the technical idea of the present disclosure. The protection scope of the present disclosure is determined only by the matters described in the claims, and embodiments that are improved and modified without departing from the gist of the present disclosure will be considered to fall within the protection scope of the present disclosure as long as they are obvious to a person having ordinary knowledge in the technical field to which the present disclosure belongs.

## Claims

1. A multimodal biosignal-based avatar lip-sync animation generation device comprising:
a multimodal data collection circuit configured to collect multimodal data including biosignal data which includes brain waves emitted from a user, including speaking and image data;
a preprocessing circuit configured to preprocess the multimodal data;
a feature extraction circuit configured to extract feature vectors including a biosignal feature and facial feature of the user from the preprocessed multimodal data;
an avatar generation circuit configured to generate an avatar that represents an appearance of the user;
a lip-sync reconstruction circuit configured to predict a mouth shape and facial movement after the multimodal data is collected, by inputting the extracted feature vectors into a pre-prepared lip-sync reconstruction model; and
a lip-sync animation implementation circuit configured to implement an avatar lip-sync animation by applying the mouth shape and facial movement predicted by the lip-sync reconstruction circuit to the avatar generated by the avatar generation circuit.

2. The device according to claim 1, wherein the avatar generation circuit is configured to generate an avatar in a two-dimensional or three-dimensional form from the image data of the user using computer vision technology, and maps the facial feature extracted by the feature extraction circuit to the generated avatar to thereby specify a facial landmark; and
the lip-sync animation implementation circuit is configured to implement an avatar lip-sync animation by applying the mouth shape and facial movement predicted by the lip-sync reconstruction circuit to the avatar generated by the avatar generation circuit, based on the coordinate values of the facial landmark.

3. The device according to claim 1 or 2, further comprising a feature convergence circuit configured to converge the feature vectors extracted from the feature extraction circuit and converting them into an embedding convergence vector, wherein the lip-sync reconstruction circuit is configured to predict mouth shape and facial movement by inputting the embedding convergence vector into the pre-prepared lip-sync reconstruction model.

4. The device according to any one of claims 1 to 3, wherein the multimodal data collection circuit includes:
a presented sentence transfer display circuit configured to transfer a presented sentence to the user;
a biosignal collection circuit configured to collect biosignal data by measuring biosignals including brain waves of a user;
an image collection circuit configured to collect image data by photographing a facial image of the user; and
a data storage circuit configured to store the biosignal data of the user in response to the transferred presented sentence and the image data, together with a trigger value being recorded over time.

5. The device according to claim 4, wherein the biosignal collection circuit further includes an electromyography in the measured biosignal of the user; and wherein
the lip-sync reconstruction circuit is configured to predict the mouth shape and facial movement by inferring articulatory organ movement trajectories based on the electromyography.

6. The device according to any one of claims 3 to 5, wherein the feature convergence circuit is configured to:
apply a weight, based on a predetermined standard, to the feature vectors extracted by the feature extraction circuit; and
converge the feature vectors to which the weight has been applied and converts them into an embedding convergence vector.

7. The device according to any one of claims 1 to 6, wherein the lip-sync reconstruction model comprises of any one of:
a first prediction circuit configured to predict the mouth shape and facial movement from the extracted feature vectors, or
a second prediction circuit configured to identify and classify intentions of the user from the extracted feature vectors, and predict the mouth shape and facial movement based on the classified intentions.

8. A multimodal biosignal-based avatar lip-sync animation generation method comprising:
collecting multimodal data including biosignal data which includes brain waves emitted by the user, including speaking and image data;
preprocessing the multimodal data;
extracting feature vectors including a biosignal feature and facial feature of the user from the preprocessed multimodal data;
generating an avatar that represents an appearance of the user based on facial features among the extracted feature vectors;
predicting the mouth shape and facial movement after the multimodal data is collected by inputting the extracted feature vectors into a pre-prepared lip-sync reconstruction model; and
implementing an avatar lip-sync animation by applying the mouth shape and facial movement predicted in the lip-sync reconstruction step to the avatar generated in the avatar generation step.

9. The method according to claim 8, further comprising converging the extracted feature vectors,
wherein the lip-sync reconstruction step predicts the mouth shape and facial movement by inputting the embedding convergence vector into the pre-prepared lip-sync reconstruction model.
